Europäisches Patentamt

European Patent Office

Office européen des brevets

(19)

(11) Publication number: **0 184 300**
**A1**

(12) # EUROPEAN PATENT APPLICATION

(21) Application number: **85307402.9**

(22) Date of filing: **15.10.85**

(51) Int. Cl.⁴: **C 07 C 17/08**
**C 07 C 17/20**

(30) Priority: **25.10.84 GB 8426997**

(43) Date of publication of application:
**11.06.86 Bulletin 86/24**

(84) Designated Contracting States:
**DE FR GB IT**

(71) Applicant: **IMPERIAL CHEMICAL INDUSTRIES PLC**
**Imperial Chemical House Millbank**
**London SW1P 3JF(GB)**

(72) Inventor: **Burgess, Leslie**
**1 Poolside Road**
**Runcorn Cheshire(GB)**

(72) Inventor: **Maling, Guy Quentin**
**Church Croft Horton Tilston Cape**
**Malpas Cheshire(GB)**

(74) Representative: **Stephenson, Kenneth et al,**
**Imperial Chemical Industries PLC Legal Department:**
**Patents PO Box 6 Bessemer Road**
**Welwyn Garden City Herts, AL7 1HD(GB)**

(54) **Process for the preparation of bromotrifluoromethane.**

(57) A method for the preparation of bromotrifluoromethane which comprises contacting bromochlorodifluoromethane with a fluorination catalyst at a temperature in the range 100-550°C.

EP 0 184 300 A1

Croydon Printing Company Ltd

CHEMICAL PROCESS

This invention relates to a chemical process and more particularly to a method for the preparation of bromotrifluoromethane.

Bromotrifluoromethane is a known chemical compound useful as a fire-extinguishing agent and as a refrigerant. It is usually manufactured by the bromination of trifluoromethane as described, for example, in German Patent 1 155 104. This method suffers from the disadvantages that trifluoromethane requires special storage apparatus because of its very low boiling point and that special materials of construction are required for the reactor in order to avoid severe corrosion by the bromine at the high reaction temperature (650-800°C). When chlorine is included in the reaction mixture to lower the bromination temperature as described in United States Patent 2658086, a substantial amount of chlorotrifluoromethane is produced as by-product reducing the yield of bromotrifluoromethane to 36.6 mole per cent.

Another method that has been proposed in German Offenlegungsschrift 1913405 is to contact dibromodifluoromethane in a solvent with aluminium chloride or aluminium bromide at a temperature between -20 and 120°C, the dibromodifluoromethane disproportionating to form bromotrifluoromethane and tetrabromomethane.

It has now been found that bromotrifluoromethane can be prepared in good yield from bromochlorodifluoromethane by a catalytic process.

Thus, according to the invention, there is provided a method for the preparation of bromotrifluoromethane which comprises contacting bromochlorodifluoromethane with a fluorination catalyst at a temperature in the range 100-550°C.

The bromochlorodifluoromethane may be used in substantially pure form or in the form of a mixture with dibromodifluoromethane.

Fluorination catalysts are well known in the art and include the following:

1)   Iron on carbon
2)   Mixed oxide of aluminium, chromium and magnesium.
3)   Chromium oxide catalysts as described in our United Kingdom Patent specification No. 1307224, the chromium oxide optionally having been subjected to a prefluorination treatment with the hydrogen fluoride before use. Also chromium fluoride and chromium oxyfluoride. Any of these may be used on a carbon support.
4)   Alumina that has been treated with hydrogen fluoride.
5)   Aluminium fluoride alone or with alumina.
6)   Potassium, rubidium or cerium fluoride alone or with alumina or aluminium fluoride.
7)   Chromium, cobalt, nickel or thorium oxide or fluoride optionally on a support such as graphite.
8)   Thorium, zirconium or palladium fluoride on carbon.
9)   Magnesium or tungsten fluoride.
10)  Active carbon

Aluminium fluoride should be given a pre-activation treatment by heating in a stream of bromochlorodifluoromethane and/or dibromodifluoromethane, with diluent if desired, to about 350°C. Thereafter, the reaction may be carried out at the desired temperature.

The catalyst may be employed in the form of a fixed or fluidised bed of appropriate size.

The method of the invention may be conveniently performed at normal atmospheric pressure but higher or lower pressures may be used if desired. An inert gaseous diluent, for example nitrogen or helium, may be present if desired.

The gaseous reaction product consists largely of bromotrifluoromethane and chlorotrifluoromethane. The proportion of the desired bromotrifluoromethane in the product may be increased by including hydrogen fluoride in the gas feed.

Thus, a preferred embodiment of the invention comprises contacting a gaseous mixture of bromochlorodifluoromethane and hydrogen fluoride and optionally dibromodifluoromethane with a catalyst at a temperature in the range 200-350°C.

The molar ratio of hydrogen fluoride to bromochlorodifluoromethane and any dibromodifluoromethane in the gas feed is suitably in the range 0.1-5 : 1, especially about 1:1.

The selectivity to bromotrifluoromethane may also be improved by including hydrogen bromide in the gas feed, especially when hydrogen fluoride is also present. The molar ratio of hydrogen bromide to bromochlorodifluoromethane and any dibromodifluoromethane in the gas feed is suitably in the range 0.1-5:1, especially about 1:1.

Although the method of the invention may be carried out in a batchwise manner, it is preferably carried out continuously. Conventional means may be used to recover the reaction product and to isolate the bromotrifluoromethane from any by-products.

The invention is illustrated but not limited by the following Examples:

Examples 1-11

The reactions were performed in a tubular reactor heated by an electric furnace. Off-gases from the reactor were passed through (1) a catchpot at about 30°C, (2) an acid scrubber containing 25% potassium hydroxide solution at about 40°C and (3) a liquid nitrogen receiver at -198°C. The gases leaving the scrubber were analysed by glc.

In those Examples using aluminium fluoride as catalyst, the dry aluminium fluoride (KAF 1000) specially prepared for use in fluidised beds and having a mean particle size of 150um was charged to the reactor whilst maintaining a helium gas flow of 200 $cm^3$/min to assist fluidisation of the catalyst. The aluminium fluoride was activated by passing a stream of bromochlorodifluoromethane through it and raising the temperature to 350°C. The catalyst was then cooled to the desired reaction temperature.

In Examples 9 and 10 the aluminium fluoride was used as a fixed bed.

In those Examples using chromia as catalyst, the pelletised catalyst, prepared as described in our United Kingdom Patent Specification No. 1407224, was charged to the reactor and heated to 350°C in a helium gas stream of 200 cm$^3$/min to drive off moisture. The catalyst was then fluorinated in a stream of hydrogen fluoride.

The bromochlorodifluoromethane, hydrogen fluoride, hydrogen bromide and helium gases were each metered into the reactor through a calibrated rotameter. The helium flow was 200 cm$^3$/min in each Example.

Further details are given in Table 1.

The reactor was a vertically disposed tube of "Inconel" metal having a bore of 5cm and a length of 85 cm, there being a 2.5 cm tip to remove spent catalyst and two gas inlet tubes at the lower end. A disengagement space at the top of the tube prevented fine catalyst particles being elutriated into the outlet lines and a 1 cm diameter thermocouple pocket running down the centre of the reactor enabled the temperature of the catalyst to be measured. The reactor tube was heated by an electronically controlled furnace. Trace heating and lagging were provided at the bottom of the reactor and the top disengagement space to prevent the condensation of organic materials.

## TABLE 1

| EX | Catalyst | Vol cc | CF$_2$BrCl cc/min | total (gm) | HF cc/min | gm total | HBr cc/min | gm total | TRAPS (g) 1 | 2 | 3 | ANALYSIS % A | B | C | D | E | TEMP RANGE | %Y A |
|----|----------|--------|-------------------|------------|-----------|----------|------------|----------|-------------|----|----|-------------|----|----|----|----|-----------|------|
| 1 | AlF$_3$ | 800 | 290 | 340 | – | – | – | – | 82 | 63 | 170 | 44 | 28 | 7 | 2 | 17 | 115–125 | 27 |
| 2 | AlF$_3$ | 800 | 290 | 309 | – | – | 320 | 165 | 108 | 105 | 174 | 54 | 30 | 9 | – | 3 | 250–270 | 38 |
| 3 | Chromia | 900 | 290 | 307 | – | – | – | – | 44 | 26 | 210 | 41 | 34 | 7 | 3 | 12 | 230–270 | 35 |
| 4 | AlF$_3$ | 800 | 290 | 315 | 310 | 40 | – | – | 2 | 120 | 221 | 43 | 52 | 4 | – | 1 | 320–340 | 40 |
| 5 | AlF$_3$ | 800 | 150 | 165 | 160 | 21 | 300 | 161 | 31 | 211 | 110 | 65 | 24 | 5 | – | 1 | 265–272 | 51 |
| 6 | AlF$_3$ | 800 | 290 | 288 | 320 | 37 | 320 | 151 | 2 | 230 | 228 | 57 | 40 | 3 | – | – | 310–340 | 56 |
| 7 | Chromia | -900 | 290 | 426 | 420 | 75 | – | – | 7 | 141 | 317 | 52 | 46 | – | – | – | 230–275 | 50 |
| 8 | Chromia | 900 | 290 | 311 | 320 | 42 | 320 | 167 | 15 | 264 | 241 | 69 | 29 | 2 | – | – | 255–265 | 65 |
| 9 | AlF$_3$ | 800 | 290 | 298 | 325 | 41 | – | – | 3 | 108 | 221 | 40 | 57 | 2 | – | 1 | 325–350 | 40 |
| 10 | AlF$_3$ | 800 | 290 | 264 | 330 | 36 | 330 | 152 | 5 | 206 | 219 | 52 | 44 | 3 | – | 1 | 340–355 | 54 |
| 11 | Active Carbon | 800 | 290 | 238 | 275 | 32 | 275 | 131 | 33 | 198 | 161 | 52 | 35 | 5 | 1 | 7 | 520–535 | 44 |

KEY : Trap 1 is catchpot

Trap 2 is scrubber

Trap 3 is liquid N2 receiver

A is CF$_3$Br

B is CF$_3$Cl

C is CF$_2$BrCl

D is CF$_2$Br$_2$

E is CF$_2$Cl$_2$

%Y is conversion of fed material to CF3Br

Example 12-14

These Examples illustrate the use of mixtures of bromochlorodifluoromethane and dibromodifluoromethane using 800 cm$^3$ of an aluminium fluoride catalyst and a helium flow of 200 cm$^3$/min.

Further details are given in Table 2.

TABLE 2

| Ex | $CF_2Br_2$ | | $CF_2BrCl$ | | HF | | TRAPS(g) | | | ANALYSIS% | | | | | TEMP | %Y |
|----|------|---------|------|---------|--------|---------|----|-----|-----|----|----|---|---|---|---------|----|
|    | cc/min | total(g) | cc/min | total(g) | cc/min | total(g) | 1 | 2 | 3 | A | B | C | D | E | RANGE | A |
| 12 | .94 | 300 | 92 | 103 | 360 | 49 | 6 | 155 | 300 | 75 | 20 | 1 | 3 | – | 360–380 | 75 |
| 13 | .65 | 215 | 150 | 172 | 360 | 50 | 6 | 146 | 299 | 65 | 29 | 2 | 3 | – | 360–380 | 64 |
| 14 | .38 | 120 | 248 | 277 | 360 | 50 | 12 | 142 | 295 | 52 | 43 | 2 | 2 | – | 343–370 | 49 |

KEY : Trap 1 is catchpot

Trap 2 is scrubber

Trap 3 is liquid N2 receiver

A is $CF_3Br$

B is $CF_3Cl$

C is $CF_2BrCl$

D is $CF_2Br_2$

E is $CF_2Cl_2$

%Y is conversion of fed material to $CF_3Br$

The $CF_2Br_2$ flow is cc/min of <u>liquid.</u>

CLAIMS

1.     A method for the preparation of bromotrifluoromethane which comprises contacting bromochlorodifluoromethane with a fluorination catalyst at a temperature in the range 100-550°C.

2.     A method according to claim 1 wherein the bromochlorodifluoromethane is used in the form of a mixture with dibromodifluoromethane.

3.     A method according to claim 1 wherein the fluoroination catalyst is selected from aluminium fluoride, chromia and active carbon.

4.     A method according to claim 1 wherein a gaseous mixture of bromochlorodifluoromethane and hydrogen fluoride is contacted with the catalyst at a temperature in the range 200-350°C.

5.     A method according to claim 1 wherein hydrogen bromide is included in the gas feed.

European Patent
Office

EUROPEAN SEARCH REPORT

0184300
Application number

EP 85 30 7402

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| | No relevant documents have been disclosed ----- | | C 07 C 17/20 |

| | |
|---|---|
| | TECHNICAL FIELDS SEARCHED (Int. Cl.4) |
| | C 07 C 19/00<br>C 07 C 17/00 |

The present search report has been drawn up for all claims

| Place of search THE HAGUE | Date of completion of the search 06-03-1986 | Examiner VAN GEYT J.J.A. |
|---|---|---|